# EUROPEAN PATENT APPLICATION

(11) **EP 4 611 264 A1**
(43) Date of publication of application: **03.09.2025**
(21) Application number: 25160507.7
(22) Date of filing: 27.02.2025
(51) Int. Cl.: H03M 7/30

(54) **METHOD AND APPARATUS FOR DECOMPRESSING DATA, ELECTRONIC DEVICE, AND COMPUTER-READABLE STORAGE MEDIUM**

(30) Priority: 27.02.2024 CN 202410216882
(71) Applicant: GeneMind Biosciences Company Limited, Shenzhen City, 518023 Guangdong (CN)
(72) Inventor: LI, Linsen, Shenzhen City, 518023 (CN)
(74) Representative: Schiweck Weinzierl Koch Patentanwälte Partnerschaft mbB

(57) **Abstract**

The present application provides a method for decompressing data, an apparatus for decompressing data, an electronic device, and a computer-readable storage medium. The method for decompressing data includes: a) acquiring compressed data, the compressed data including a plurality of compression blocks connected in series, one compression block including a header and a data main body connected to the header, and the header having an identifiable feature; b) identifying the identifiable feature to position a corresponding header, and determining one or more to-be-decompressed blocks in the compressed data according to the positioned header, one to-be-decompressed block including one or more compression blocks; and c) decompressing the to-be-decompressed block by a single thread or a plurality of threads so as to obtain decompressed compressed data, where b) and c) are performed in parallel. The data decompression mode has high efficiency, and can effectively shorten the decompression time especially under the condition that huge data needs to be decompressed.

## Description

### TECHNICAL FIELD

The present disclosure relates to the field of data processing technologies, and in particular, to a method for decompressing data, an apparatus for decompressing data, an electronic device, and a computer-readable storage medium.

### BACKGROUND

In the related art, data compression and decompression are common data processing modes. Data compression means that on the premise of not losing useful information, the data size is reduced to reduce the memory space, and the transmission, storage and processing efficiency is improved. Data decompression is a processing mode corresponding to data compression, and when corresponding data is needed, the data is decompressed from compressed data.

When decompressing the data, a single thread or a plurality of threads are widely used to decompress the data, and one or more different threads are operated to execute the task of decompressing the data. However, whether the single thread or the plurality of threads are adopted, the improvement of the decompression efficiency is always an objective to be achieved by the technical personnel in the art.

### SUMMARY

In view of this, embodiments of the present disclosure provide a method for decompressing data, an apparatus for decompressing data, an electronic device, and a computer-readable storage medium. An embodiment of the present disclosure provides a method for decompressing data, including:
a) acquiring compressed data, where the compressed data includes a plurality of compression blocks connected in series, one compression block includes a header and a data main body connected to the header, and the header has an identifiable feature;
b) identifying the identifiable feature to position a corresponding header, and determining one or more to-be-decompressed blocks in the compressed data according to the positioned header, where one to-be-decompressed block includes one or more compression blocks; and
c) decompressing the to-be-decompressed block by a single thread or a plurality of threads so as to obtain decompressed compressed data,
where b) and c) are performed in parallel.

According to the method for decompressing data of the present application, the compressed data is obtained firstly, then the header of the compression block is positioned by identifying the identifiable feature, the to-be-decompressed block of the compressed data is determined according to the positioned header, and finally the determined to-be-decompressed block is decompressed by the single thread or the plurality of threads so as to obtain the decompressed compressed data. The data decompression mode has high efficiency, and can effectively shorten the decompression time especially under the condition that huge data needs to be decompressed.

An embodiment of the present disclosure provides an apparatus for decompressing data, including: a data acquisition unit, configured to perform a), acquiring compressed data, where the compressed data includes a plurality of compression blocks connected in series, one compression block includes a header and a data main body connected to the header, and the header has an identifiable feature;
a block determination unit, configured to perform b), identifying the identifiable feature to position a corresponding header, and determining one or more to-be-decompressed blocks in the compressed data according to the positioned header, where one to-be-decompressed block includes one or more compression blocks; and
a decompression unit, configured to perform c), decompressing the to-be-decompressed block by a single thread or a plurality of threads so as to obtain decompressed compressed data,
where b) and c) are performed in parallel.

An embodiment of the present disclosure provides an electronic device, including a memory, configured to store data and including a computer executable program; and a processor, configured to execute the computer executable program to implement the method for decompressing data mentioned above.

An embodiment of the present disclosure provides a computer-readable storage medium, configured to store a program executed by a computer, where executing the program includes implementing the method for decompressing data mentioned above.

The additional aspects and advantages of the embodiments of the present application will be partially set forth in the following description, and will partially become apparent from the following description or be appreciated by practice of the present application.

### BRIEF DESCRIPTION OF THE DRAWINGS

The above and/or additional aspects and advantages of the present disclosure will become apparent and readily understood from the following description of embodiments in conjunction with the accompanying drawings. In the accompanying drawings:
FIG. 1 is a schematic flow chart of a method for decompressing data according to some embodiments of the present disclosure;
FIG. 2 is a schematic flow chart of a method for decompressing data according to some embodiments of the present disclosure;
FIG. 3 is a schematic flow chart of a method for decompressing data according to some embodiments of the present disclosure;
FIG. 4 is a schematic flow chart of a method for decompressing data according to some embodiments of the present disclosure;
FIG. 5 is a schematic diagram of determining a header according to some embodiments of the present disclosure;
FIG. 6 is a schematic diagram of determining a header according to some embodiments of the present disclosure; and
FIG. 7 is a module schematic diagram of an apparatus for decompressing data according to some embodiments of the present disclosure.

### DETAILED DESCRIPTION

The embodiments of the present application are described in detail hereinafter, with examples of the embodiments illustrated in the accompanying drawings. The embodiments described below with reference to the accompanying drawings are exemplary and are intended to explain the present application, rather than being construed as limiting the present application.

In the process of describing the present application, the terminology herein is explained and illustrated in the drawings only for the purpose of facilitating an understanding of the solution and is not to be construed as limiting the protection solution of the present application.

As used herein, the singular forms "a", "an", and the like, include plural referents (one or more) unless otherwise indicated; and "a set of " or "a plurality of" refers to two or more.

As used herein, unless otherwise specified, the term "include", or "comprise" is open-ended and does not exclude the inclusion of other contents or situations that are consistent with the stated situations but not listed or exemplified herein.

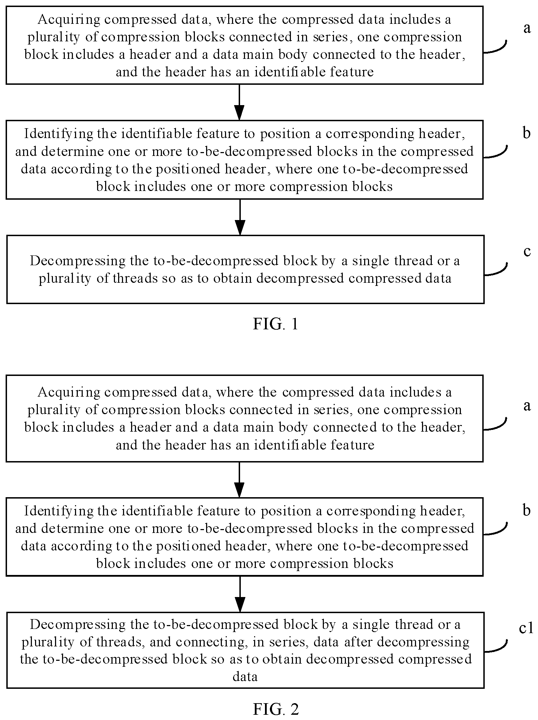

Referring to FIG. 1, an embodiment of the present application provides a method for decompressing data, including:
a) acquiring compressed data, where the compressed data includes a plurality of compression blocks connected in series, one compression block includes a header and a data main body connected to the header, and the header has an identifiable feature;
b) identifying the identifiable feature to position a corresponding header, and determining one or more to-be-decompressed blocks in the compressed data according to the positioned header, where one to-be-decompressed block includes one or more compression blocks; and
c) decompressing the to-be-decompressed block by a single thread or a plurality of threads so as to obtain decompressed compressed data,
where b) and c) are performed in parallel.

In the present embodiment, the compressed data may be in a format of rar, zip, gzip, etc., and the method for decompressing data of the present application is used to decompress compressed data in various formats.

For example, compressed data in a gzip format is described in detail herein, the compressed data in the gzip format includes a plurality of compression blocks connected in series, each compression block includes a header and a data main body connected to the header, and at least part of the compression blocks may further include a tail portion connected to the data main body.

Regarding the compressed data in the gzip format, the compression blocks included therein are constructed as follows (see specifically https://blog.csdn.net/ljx333/article/details/77586147):

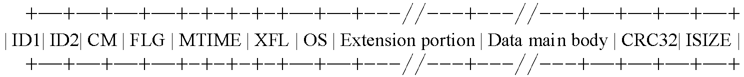

For each header, the header stores basic information of the compression block, including a data type, version, size, creation time, modification time, access right, etc., which has the function of helping an operating system identify the data type and access right. The header consists of a fixed-length portion and an extension portion, the fixed-length portion is the first ten bytes of the compression block, and the first ten bytes are as follows:
ID 1 and ID2 each occupy 1 byte, and ID1 and ID2 are used to identify compression blocks in a gzip format, e.g., ID1 = 31 (0 × 1F), and ID2 = 139 (0 × 8B).

CM occupies 1 byte, and CM is used to identify a compression method used by a current compression block, e.g., CM = 8, indicating that the compression block employs a DEFLATE method.

FLG occupies 1 byte, and is a flag bit, where each bit in the flag bit represents whether a corresponding extension bit exists later, and the meaning of each bit is as follows: if the Bit 0FTEXT is set, it indicates that the compression block is ASCII text data; if Bit 1FHCRC is set, it indicates that the CRC16 check is performed on the header; if the Bit 2FEXTRA is set, it indicates that the extension portion exists; if the Bit 3FNAME is set, it indicates that a name field of compressed original data exists; if the Bit 4FCOMMENT is set, it indicates that a comment field exists; and Bits 5-7 are reserved and all set as 0.

MTIME occupies 4 bytes, and is used to represent the time when the compressed original data was last modified, the time being in a UINX format.

XFL occupies 1 byte, and is used to identify a compression method, and since the gzip format has only the DEFLATE method, aiming at the DEFLATE method, XFL has the following meaning: XFL = 2, which represents that the compression ratio is maximum but the compression speed is lowest; and XFL = 4, which represents that the compression speed is highest.

OS occupies 1 byte, and represents a file system to be compressed.

The fixed-length portion necessarily exists, the extension portion does not necessarily exist, and whether the extension portion exists or not is determined by the fixed-length portion. If the extension portion exists, the extension portion is divided into four parts, which are as follows in order (the order cannot be exchanged): FEXTRA + FNAME + FCOMMENT + FHCRC.

For the data main body, a quantity of bytes occupied by the data main body is determined according to the compressed original data.

For the tail portion, the tail portion occupies 4 bytes, CRC32 represents a result of calculating the compressed original data by using a standard redundancy check algorithm, and ISIZE represents a result of modulo 2^32 by the size of the compressed original data. In some embodiments, the compression block may contain only a header and a data main body, with no tail portion.

The thread is the smallest unit for the operation scheduling of the operating system, and belongs to the actual operation unit in a course. One thread refers to a single sequential control flow in the course, one course can concurrently run a plurality of threads, and each thread concurrently executes different tasks. According to the method for decompressing data of the present application, the to-be-decompressed block may be decompressed by the single thread, or the to-be-decompressed block may be decompressed by the plurality of threads, where a quantity of the plurality of threads is, for example, 4, 5, and 6, which is specifically configured according to actual situations, and is not limited herein.

According to the method for decompressing data of the present application, specifically, a) is performed, the compressed data is obtained from a memory space, the compressed data includes the plurality of compression blocks connected in series, each compression block includes a header and a data main body connected to the header, and the header has an identifiable feature. For example, in the field of sequencing, a large amount of sequencing data such as fq data is compressed, in order to compress the data, and the compressed data may be obtained from the memory space, where the memory space may be a memory which is disposed inside a sequencer and used to store the compressed data or a memory which is connected to the sequencer externally and used to store the compressed data.

Then, b) is performed, the identifiable feature is identified in the compressed data to position the corresponding header, and the specific identification process may start from the initial position of the compressed data and end at the middle position or the end position, and the corresponding header is positioned every time the feature the same as the identifiable feature is identified. After the header is positioned, one or more to-be-decompressed blocks in the compressed data are determined according to the header, where each to-be-decompressed block contains one or more compression blocks.

The middle position or the end position mentioned above is determined according to the requirement of decompressing the whole compressed data or part of the compressed data. For example, if the whole compressed data needs to be decompressed, the identification process starts from the initial position of the compressed data and ends at the end position; and if the part of the compressed data needs to be decompressed, the identification process starts from the initial position of the compressed data and ends at the middle position of the whole compressed data, and the middle position of the whole compressed data corresponds to the end position of the part of the compressed data needing to be decompressed. The middle position refers to any position from the initial position to the end position of the compressed data, and does not refer to a position of half the size of the compressed data.

In other embodiments, it may be realized that the compressed data is identified from a first middle position of the compressed data to a second middle position or the end position, and the identification process is as described above.

The identifiable feature consists of at least 1 byte, and identifying the identifiable feature refers to identifying the identifiable feature by identifying the at least 1 byte. If the identifiable feature consists of a plurality of bytes, the identifiable feature may be identified by identifying 1 byte in the plurality of bytes and further identifying the rest bytes; or the identifiable feature may be identified by identifying all of the plurality of bytes.

After one or more to-be-decompressed blocks are determined, c) is performed, and the to-be-decompressed block is decompressed by the single thread or the plurality of threads so as to obtain the decompressed compressed data. A single thread decompression mode is adopted, and the determined to-be-decompressed blocks are decompressed one by one by the single thread. A multi-thread decompression mode is adopted, after the to-be-decompressed block is determined, if there is a thread in an idle state, the thread in the idle state is called to decompress the determined to-be-decompressed block, and if there is no thread in the idle state, it waits to call the thread in the idle state until there is the thread in the idle state, and then the thread in the idle state is called to decompress the determined to-be-decompressed block.

In the present embodiment, b) and c) mentioned above are performed in parallel.

Specifically, after one to-be-decompressed block or the plurality of to-be-decompressed blocks are determined, the thread in the idle state may be called to decompress the determined to-be-decompressed blocks, and compared to decompressing the to-be-decompressed blocks after all the to-be-decompressed blocks in the compressed data are determined, the decompression efficiency can be effectively improved.

For the method for decompressing data, for example, the compressed data includes five compression blocks A, B, C, D, and E connected in series, and the plurality of threads are three threads a, b, and c. After the compressed data is obtained, starting from the initial position of the compressed data, a to-be-decompressed block A1 is determined, the to-be-decompressed block includes the compression block A, at this time, the threads a, b, and c are all in the idle state, and the thread a is called to decompress the to-be-decompressed block A1. Then a to-be-decompressed block B1 is determined, the to-be-decompressed block B1 includes the compression block B, at this time, the thread a is still decompressing the to-be-decompressed block A1, the threads B and c are both in the idle state, and the thread B is called to decompress the to-be-decompressed block B1. Then a to-be-decompressed block C1 is determined, the to-be-decompressed block C1 includes the compression block C, the thread a is still decompressing the to-be-decompressed block A1, the thread B is still decompressing the to-be-decompressed block B1, the thread C is in the idle state, and the thread C is called to decompress the to-be-decompressed block C1. Then a to-be-decompressed block D1 is determined, the to-be-decompressed block D1 includes the compression block D, at this time, the thread a completes decompression of the to-be-decompressed block A1 and is in the idle state, the thread B is still decompressing the to-be-decompressed block B1, the thread C is still decompressing the to-be-decompressed block C1, and the thread a is called to decompress the to-be-decompressed block D1. Then a to-be-decompressed block E1 is determined, the to-be-decompressed block E1 includes the compression block E, at this time, the thread b completes decompression of the to-be-decompressed block B1 and is in the idle state, the thread a is still decompressing the to-be-decompressed block D1, the thread c is still decompressing the to-be-decompressed block C1, and the thread b is called to decompress the to-be-decompressed block E1. After the thread a completes decompression of the to-be-decompressed block D1, the thread B completes decompression of the to-be-decompressed block E1 and the thread C completes decompression of the to-be-decompressed block C1, the to-be-decompressed blocks A1, B1, C1, D1, and E1 are all decompressed, such that the decompressed compressed data is obtained.

According to the method for decompressing data of the present application, the compressed data is obtained firstly, then the header of the compression block is positioned by identifying the identifiable feature, the to-be-decompressed block of the compressed data is determined according to the positioned header, and finally the determined to-be-decompressed block is decompressed by the single thread or the plurality of threads so as to obtain the decompressed compressed data. The data decompression process has high efficiency, and can effectively shorten the decompression time especially under the condition that huge data needs to be decompressed.

Referring to FIG. 2, in some specific embodiments of the present application, c) further includes c1) connecting, in series, data after decompressing the to-be-decompressed blocks so as to obtain the decompressed compressed data.

Specifically, after the decompression of the to-be-decompressed blocks is completed, the data obtained by decompression is connected in series, such that the decompressed compressed data is obtained.

In some embodiments, every time decompression of one to-be-decompressed block is completed, the data obtained by decompression is connected according to a serial sequence. For example, a first to-be-decompressed block, a second to-be-decompressed block, and a third to-be-decompressed block are determined, and the serial sequence of the first to-be-decompressed block, the second to-be-decompressed block, and the third to-be-decompressed block is consistent with a serial sequence of the compression blocks contained therein. If the compression block contained in the second to-be-decompressed block is located between the compression block contained in the first to-be-decompressed block and the compression block contained in the third to-be-decompressed block in the serial sequence, after the first to-be-decompressed block and the second to-be-decompressed block are decompressed, the data obtained by decompression is connected in series, and after the third to-be-decompressed block is decompressed, the data after decompressing the third to-be-decompressed block is connected in series to the data connected in series after decompressing the first to-be-decompressed block and the second to-be-decompressed block. If the compression block contained in the third to-be-decompressed block is located between the compression block contained in the first to-be-decompressed block and the compression block contained in the second to-be-decompressed block in the serial sequence, the data obtained by decompression is connected in series according to the sequence of the first to-be-decompressed block, the third to-be-decompressed block, and the second to-be-decompressed block after the third to-be-decompressed block is decompressed even if the first to-be-decompressed block and the second to-be-decompressed block are decompressed firstly.

In some embodiments, after decompression of all the to-be-decompressed blocks is completed, the data obtained by decompression is connected according to the serial sequence. For example, decompression of all to-be-decompressed blocks such as the first to-be-decompressed block, the second to-be-decompressed block, and the third to-be-decompressed block is completed, and the serial sequence of the first to-be-decompressed block, the second to-be-decompressed block, and the third to-be-decompressed block is consistent with a serial sequence of the compression blocks contained therein. If the compression block contained in the second to-be-decompressed block is located between the compression block contained in the first to-be-decompressed block and the compression block contained in the third to-be-decompressed block in the serial sequence, the data obtained by decompression is connected in series according to the sequence of the first to-be-decompressed block, the second to-be-decompressed block, and the third to-be-decompressed block after decompression of the first to-be-decompressed block, the second to-be-decompressed block, and the third to-be-decompressed block is completed. If the compression block contained in the third to-be-decompressed block is located between the compression block contained in the first to-be-decompressed block and the compression block contained in the second to-be-decompressed block in the serial sequence, the data obtained by decompression is connected in series according to the sequence of the first to-be-decompressed block, the third to-be-decompressed block, and the second to-be-decompressed block after decompression of the first to-be-decompressed block, the second to-be-decompressed block, and the third to-be-decompressed block is completed.

In order to avoid the situation that the decompressed compressed data and the compressed original data have data non-correspondence, when c1) connecting, in series, data after decompressing the to-be-decompressed blocks is performed, no matter a mode that the data obtained by decompression is connected in series according to the serial sequence every time decompression of one to-be-decompressed block is completed, or a mode that the data obtained by decompression is connected in series according to the serial sequence after decompression of all the to-be-decompressed blocks is completed is adopted, the serial sequence is associated with the serial sequence of the compression blocks contained in the to-be-decompressed blocks, but not associated with a decompression completion sequence of the to-be-decompressed blocks. For example, the obtained compressed data includes the plurality of compression blocks A, B, C, D, and E connected in series, and then the to-be-decompressed blocks A1, B1, C1, D1, and E1 are determined from the compressed data, where the to-be-decompressed block A1 includes the compression block A, the to-be-decompressed block B1 includes the compression block B, the to-be-decompressed block C1 includes the compression block C, the to-be-decompressed block D1 includes the compression block D, and the to-be-decompressed block E1 includes the compression block E. After decompression of the to-be-decompressed blocks A1, B1, C1, D1, and E1 is completed, the data obtained by decompression is connected in series according to the sequence of the to-be-decompressed blocks A1, B1, C1, D1, and E1, and the decompressed compressed data is obtained.

The above series connection refers to that the initial position of the data obtained by decompressing a current to-be-decompressed block is connected to the end position of the data obtained by decompressing a previous to-be-decompressed block, and the initial position of the data obtained by decompressing a next to-be-decompressed block is connected to the end position of the data obtained by decompressing the current to-be-decompressed block. The initial position obtained by decompressing the first to-be-decompressed block according to the serial sequence is used as the initial position of the decompressed compressed data, and the end position obtained by decompressing the last to-be-decompressed block according to the serial sequence is used as the end position of the decompressed compressed data.

In some specific embodiments of the present application, decompression is performed by the plurality of threads.

Specifically, after the to-be-decompressed blocks in the compressed data are determined, decompression is performed by the plurality of threads, decompression of the plurality of to-be-decompressed blocks can be performed simultaneously by using the plurality of threads, and the decompression efficiency is improved.

In some specific embodiments of the present application, the identifiable feature of the header is shown in the header as a portion with both size and structural features being fixed, such as the above fixed-length portion of the header, but the fixed-length portion of the header is a compression block of the compressed data corresponding to the gzip format, and for other formats, such as rar and zip formats, the identifiable feature of the header may be different from that in the gzip format. According to the method for decompressing data of the present application, the portion (such as the fixed-length portion in the gzip format) of the header is adopted as the identifiable feature, the header does not need to be modified, and the method is applied easily and conveniently and can be widely applied to various application environments.

The fixed portion consists of at least 1 byte, and can be identified by identifying the at least 1 byte. If the fixed portion consists of a plurality of bytes, the fixed portion may be identified by identifying 1 byte in the plurality of bytes and further identifying the rest bytes; or the fixed portion may be identified by identifying all of the plurality of bytes in the fixed portion.

The compressed data further has at least one of the following features (i) to (iii):
(i) the compressed data is in a gzip format, and/or an extension name of the compressed data includes one of .GZIP, .gz, or .tgz, and/or the compressed data is obtained by compressing specified data through a DEFLATE algorithm-based compression algorithm, and/or the compressed data is obtained by compressing specified data through a gzip algorithm;
(ii) the portion is connected to the data main body; and/or optionally, the header further includes an extension portion connected to the portion, and the portion is connected to the data main body through the extension portion; and
(iii) the compression block further includes a tail portion connected to the data main body.

The DEFLATE algorithm mentioned herein is a lossless data compression algorithm composed of an LZ77 algorithm and Huffman Coding, and has been widely applied to processing of various storage data.

In some embodiments, the compressed data includes feature (i). In some embodiments, the compressed data includes feature (ii). In some embodiments, the compressed data includes feature (iii). In some embodiments, the compressed data includes features (i) and (ii). In some embodiments, the compressed data includes features (i) and (iii). In some embodiments, the compressed data includes features (ii) and (iii). In some embodiments, the compressed data includes features (i) to (iii).

In some embodiments, the identifiable feature of the header is not a portion of the header itself, but is an additionally added portion, for example, an additional byte is designed in the header as the identifiable feature, and the additional byte may be obtained by following a certain regular or irregular design, is distinguished from other bytes in the compression block, and can also be applied to the method for decompressing data of the present application.

In some specific embodiments of the present application, block compression is performed on the specified data so as to obtain the compressed data; optionally, block compression is performed by the DEFLATE algorithm-based compression algorithm, and the compression algorithm optionally is a gzip algorithm; and optionally, block compression is performed by the single thread or the plurality of threads.

Specifically, block compression is performed on the basis of the specified data, such as sequencing data, such that the compressed data is obtained, the compressed data including a plurality of compression blocks connected in series. After obtaining the compressed data, the above specified data is equal to the above compressed original data.

Block compression may be performed by the DEFLATE algorithm-based compression algorithm. The compression algorithm may be the gzip algorithm.

Block compression is performed by the single thread or the plurality of threads so as to obtain the compressed data. A single thread compression mode is adopted, and the specified data is compressed by the single thread. A multi-thread compression mode is adopted, if there is a thread in an idle state, the thread in the idle state is called to compress the specified data, and if there is no thread in the idle state, it waits to call the thread in the idle state until there is the thread in the idle state, and then the thread in the idle state is called to compress the specified data.

In some embodiments, according to the above-mentioned memory space, the compressed data obtained by block compression on the specified data may be stored in the memory space, and when the compressed data needs to be decompressed, the compressed data is obtained from the memory space.

Here, a method for compressing data is exemplified. Original data needing to be compressed is obtained firstly, then block processing is performed on the original data to obtain a plurality of blocks, then the blocks are compressed by the single thread or the plurality of threads to obtain a plurality of compression blocks, and the plurality of compression blocks are connected in series to generate the compressed data. The generated compressed data may be stored in the memory space. For example, in the field of sequencing, a large amount of sequencing data such as fq data is compressed, in order to compress the data, and the compressed data may be stored in the memory space, where the memory space may be a memory which is disposed inside a sequencer and used to store the compressed data or a memory which is connected to the sequencer externally and used to store the compressed data.

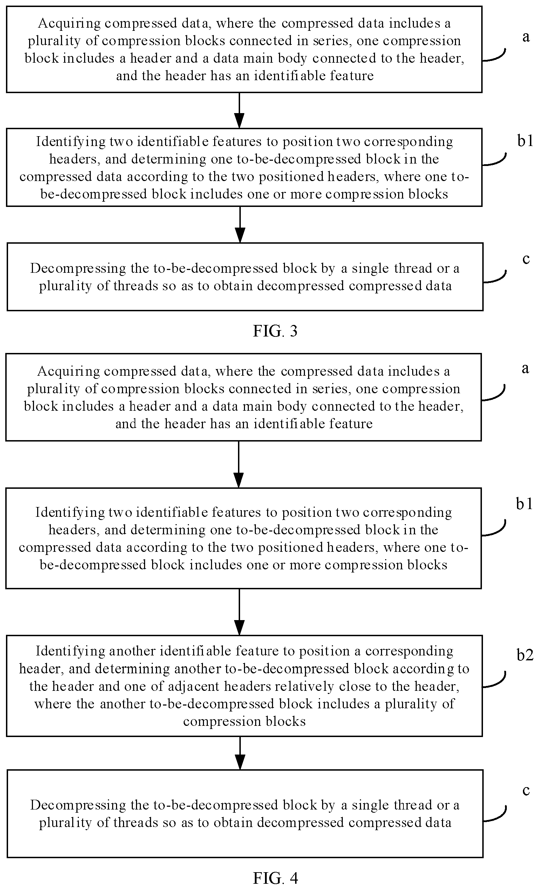

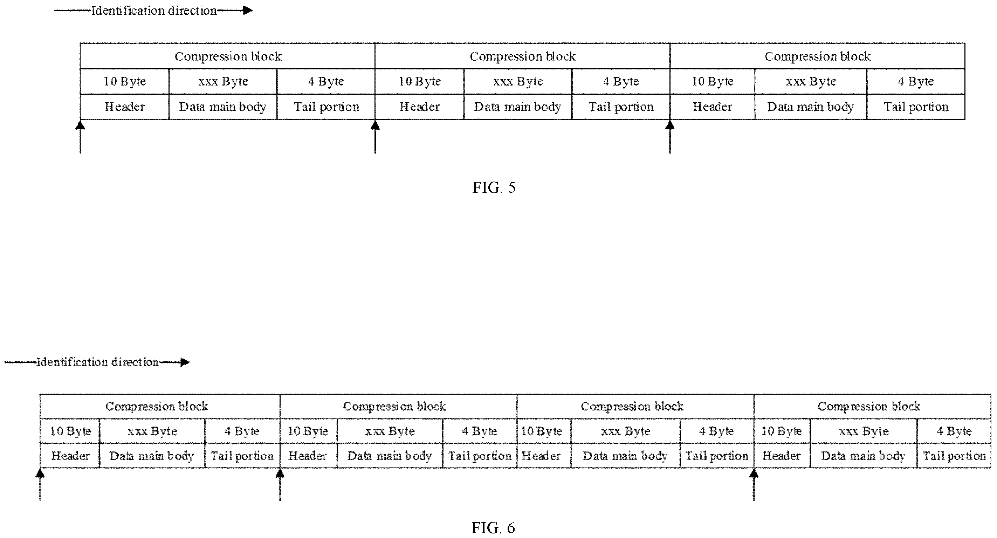

Referring to FIG. 3 and FIG. 5, in some specific embodiments of the present application, b) includes b1) identifying two identifiable features to position two corresponding headers, determining one to-be-decompressed block in the compressed data according to the two positioned headers, and enabling the to-be-decompressed block to be subjected to single-thread or multi-thread decompression of c).

Specifically, b1) is performed, the two identifiable features are identified in the compressed data, the two corresponding headers are positioned according to the two identifiable features, then one to-be-decompressed block in the compressed data is determined according to the two positioned headers, and the specific determining process is to determine the data between the two headers as one to-be-decompressed block, and then perform c) to decompress the to-be-decompressed block by the single thread or the plurality of threads. Every time b1) is performed to determine one to-be-decompressed block, c) is performed to decompress the to-be-decompressed block, such that parallel execution of b1) and c) is realized.

In some specific embodiments of the present application, the two positioned headers are adjacent headers in the compressed data.

Specifically, b1) is performed, the two identifiable features are identified in the compressed data, the two corresponding headers are positioned according to the two identifiable features, the two positioned headers are adjacent headers in the compressed data, then one to-be-decompressed block in the compressed data is determined according to the two positioned headers, the to-be-decompressed block includes one compression block, then c) is performed to decompress the to-be-decompressed block by the single thread or the plurality of threads.

In the present embodiment, the compressed data is obtained, the compressed data includes the plurality of compression blocks connected in series, which are arranged in order as a first compression block, a second compression block, a third compression block, and so on. A first identifiable feature is identified in the compressed data, a corresponding first header is positioned according to the first identifiable feature, then a second identifiable feature is identified in the compressed data, a corresponding second header is positioned according to the second identifiable feature, the first header and the second header are adjacent headers, a first to-be-decompressed block is determined according to the first header and the second header, the first to-be-decompressed block includes the first compression block, and then the first to-be-decompressed block is decompressed by the single thread or the plurality of threads. A third identifiable feature is identified in the compressed data, a third header is positioned according to the third identifiable feature, the second header and the third header are adjacent headers, a second to-be-decompressed block is determined according to the second header and the third header, the second to-be-decompressed block includes the second compression block, and the second to-be-decompressed block is decompressed by the single thread or the plurality of threads. With reference to the above, the to-be-decompressed blocks are determined and decompressed until a last identifiable feature is identified (if the identifiable feature cannot be identified later, the identified identifiable feature is the last identifiable feature), a corresponding last header is positioned according to the last identifiable feature, a last to-be-decompressed block is determined according to the last header to the end position of the compressed data, the last to-be-decompressed block includes a last compression block, the last to-be-decompressed block is decompressed by the single thread or the plurality of threads, and finally the decompressed compressed data is obtained according to all the decompressed to-be-decompressed blocks.

According to the method for decompressing data of the present application, the to-be-decompressed block is determined by taking adjacent headers as the reference, and each to-be-decompressed block includes one compression block, thereby ensuring that the compression blocks in the compressed data are not missed and ensuring the integrity of the decompressed compressed data. Moreover, the data decompression process has high efficiency, and can effectively shorten the decompression time especially under the condition that huge data needs to be decompressed. For example, in the field of sequencing, sequencing data with a huge data size needs to be decompressed, and the decompression time can be effectively shortened by the method for decompressing data. Moreover, in the field of sequencing, for sequencing data quickly generating a large amount of data in a short time or log data slowly generating a large amount of data in a long time, the method for decompressing data can effectively shorten the decompression time and also reduce the memory occupation.

In some specific embodiments of the present application, one or more headers exist between the two positioned headers.

Specifically, b1) is performed, the two identifiable features are identified in the compressed data, the two corresponding headers are positioned according to the two identifiable features, one or more headers exist between the two positioned headers, then one to-be-decompressed block in the compressed data is determined according to the two positioned headers, the to-be-decompressed block includes a plurality of compression blocks, then c) is performed to decompress the to-be-decompressed block by the single thread or the plurality of threads.

In the present embodiment, the compressed data is obtained, the compressed data includes the plurality of compression blocks connected in series. A first identifiable feature is identified in the compressed data, a corresponding first header is positioned according to the first identifiable feature, then a second identifiable feature is identified in the compressed data, a corresponding second header is positioned according to the second identifiable feature, one or more headers exist between the first header and the second header, a first to-be-decompressed block is determined according to the first header and the second header, the first to-be-decompressed block includes a plurality of compression blocks, and then the first to-be-decompressed block is decompressed by the single thread or the plurality of threads. A third identifiable feature is identified in the compressed data, a third header is positioned according to the third identifiable feature, one or more headers exist between the second header and the third header, a second to-be-decompressed block is determined according to the second header and the third header, the second to-be-decompressed block includes a plurality of compression blocks, and the second to-be-decompressed block is decompressed by the single thread or the plurality of threads. With reference to the above, the to-be-decompressed blocks are determined and decompressed until a last identifiable feature is identified (if the identifiable feature cannot be identified later, the identified identifiable feature is the last identifiable feature), a corresponding last header is positioned according to the last identifiable feature, a last to-be-decompressed block is determined according to the last header to the end position of the compressed data, the last to-be-decompressed block includes one or more compression blocks, the last to-be-decompressed block is decompressed by the single thread or the plurality of threads, and finally the decompressed compressed data is obtained according to all the decompressed to-be-decompressed blocks.

According to the method for decompressing data of the present application, the two corresponding headers are positioned according to the two identifiable features, one to-be-decompressed block in the compressed data is determined according to the two positioned headers, one or more headers exist between the two positioned headers, and the to-be-decompressed block includes the plurality of compression blocks. According to the method for decompressing data, the plurality of compression blocks are combined into one to-be-decompressed block, and then the to-be-decompressed block is decompressed, such that the decompression efficiency can be further improved, and the decompression time can be effectively shortened especially under the condition that huge data needs to be decompressed. For example, in the field of sequencing, sequencing data with a huge data size needs to be decompressed, and the decompression time can be effectively shortened by the method for decompressing data. Moreover, in the field of sequencing, for sequencing data quickly generating a large amount of data in a short time or log data slowly generating a large amount of data in a long time, the method for decompressing data can effectively shorten the decompression time and also reduce the memory occupation.

Referring to FIG. 4 and FIG. 6, in some specific embodiments of the present application, the two headers are adjacent headers in the compressed data, b) further includes b2) identifying another identifiable feature to position a corresponding header, and determining another to-be-decompressed block according to the header and one of adjacent headers relatively close to the header, and the another to-be-decompressed block includes a plurality of compression blocks.

Specifically, b) is performed firstly, the two identifiable features are identified in the compressed data, the two corresponding headers are positioned according to the two identifiable features, the two headers are adjacent headers, then one to-be-decompressed block is determined according to the two headers, the to-be-decompressed block includes one compression block, and then c) is performed to decompress the to-be-decompressed block by the single thread or the plurality of threads. b2) is performed simultaneously with c) or after c), the another identifiable feature is identified, the corresponding header is positioned according to the another identifiable feature, the another to-be-decompressed block is determined according to the header and one of the two adjacent headers relatively close to the header, the another to-be-decompressed block includes a plurality of compression blocks, and then c) is performed to decompress the another to-be-decompressed block by the single thread or the plurality of threads.

In the present embodiment, b) is performed firstly, identifiable features A1 and B1 are identified in the compressed data, corresponding headers A2 and B2 are positioned according to the identifiable features A1 and B1, the headers A2 and B2 are adjacent headers, a to-be-decompressed block a is determined according to the headers A2 and B2, the to-be-decompressed block a includes one compression block, and then c) is performed to decompress the to-be-decompressed block a by the single thread or the plurality of threads. b2) is performed simultaneously with c) or after c), another identifiable feature C1 is identified, and a corresponding header C2 is positioned according to the identifiable feature C1. If the header C2 is located in front of the headers A2 and B2 in the compressed data (the header C2 is positioned in front of the headers A2 and B2), a distance between the header C2 and the header A2 is relatively shorter than a distance between the header C2 and the header B2, a to-be-decompressed block b1 is determined according to the header C2 and the header A2, the to-be-decompressed block b1 includes a plurality of compression blocks, and then c) is performed to decompress the to-be-decompressed block b1 by the single thread or the plurality of threads. If the header C2 is located behind the headers A2 and B2 in the compressed data (the header C2 is positioned behind the headers A2 and B2), a distance between the header C2 and the header B2 is relatively shorter than a distance between the header C2 and the header A2, a to-be-decompressed block b2 is determined according to the header B2 and the header C2, the to-be-decompressed block b2 includes a plurality of compression blocks, and then c) is performed to decompress the to-be-decompressed block b2 by the single thread or the plurality of threads. According to the method for decompressing data of the present application, different identification results are integrated, different to-be-decompressed blocks are finally determined according to the different identification results, the different to-be-decompressed blocks have different quantities of compression blocks and each includes a single compression block or a plurality of compression blocks, and then the to-be-decompressed blocks are decompressed, such that the decompression efficiency can be further improved, and the decompression time can be effectively shortened especially under the condition that huge data needs to be decompressed. For example, in the field of sequencing, sequencing data with a huge data size needs to be decompressed, and the decompression time can be effectively shortened by the method for decompressing data. Moreover, in the field of sequencing, for sequencing data quickly generating a large amount of data in a short time or log data slowly generating a large amount of data in a long time, the method for decompressing data can effectively shorten the decompression time and also reduce the memory occupation.

In some specific embodiments of the present application, the compressed data is traversed in a specified step length to identify the identifiable features, the specified step length is greater than or equal to 1 byte and less than or equal to N times a size of the identifiable feature, and N is greater than or equal to 1.

Specifically, starting from the initial position of the compressed data, the identifiable features are identified in the specified step length, the corresponding header is positioned if the identifiable features are identified within the specified step length, and the header is not positioned if the identifiable features are not identified within the specified step length. The compressed data is traversed until to the end position of the compressed data needing to be decompressed. The size of the specified step length may or may not change in the traversal process.

It should be noted that, although the specified step length is less than or equal to N times the size of the identifiable feature, the specified step length is not calculated by the identifiable features, but the sizes of the specified step length and the identifiable features are only compared.

For example, starting from the initial position of the compressed data, 10 bytes are taken as the specified step length to identify the identifiable features, within each 10-byte range, whether the identifiable features are identified is judged, and if the identifiable features are identified, the corresponding header is positioned. The compressed data is traversed until to the end position of the compressed data needing to be decompressed. In the traversal process, the size of the specified step length may be changed to 9 bytes, 8 bytes or other bytes, or 10 bytes without change.

In the present embodiment, the specified step length is greater than or equal to 1 byte and less than or equal to N times the size of the identifiable feature. For example, for the compressed data in the gzip format, the size of the identifiable feature of the header of the compression block included in the compressed data is 10 bytes, so the specified step length may be defined to be greater than or equal to 1 byte and less than or equal to 10 bytes, and if N is 1, the specified step length is defined as 1 byte, 1.5 bytes, 2 bytes, 2.5 bytes, 3 bytes, 3.5 bytes, 4 bytes, 4.5 bytes, 5 bytes, 5.5 bytes, 6 bytes, 6.5 bytes, 7 bytes, 7.5 bytes, 8 bytes, 8.5 bytes, 9 bytes, 9.5 bytes, or 10 bytes. The specified step length may be changed as long as the specified byte range is met.

In some specific embodiments of the present application, the specified step length is a fixed step length, and the fixed step length takes any integer value from 1 byte to N times the size of the identifiable feature. For example, for the compressed data in the gzip format, the size of the identifiable feature of the header of the compression block included in the compressed data is 10 bytes, so the specified step length may be defined to be greater than or equal to 1 byte and less than or equal to N times 10 bytes and be an integer value, and if N is 1, the specified step length is defined as 1 byte, 2 bytes, 3 bytes, 4 bytes, 5 bytes, 6 bytes, 7 bytes, 8 bytes, 9 bytes, or 10 bytes. Although the specified step length mentioned in the present application is in units of bytes, in other embodiments, the specified step length may be in other units, such as bits, and words. The conversion relationship is that 8 bits constitute 1 byte, in a 16-bit computer, 2 bytes are 1 word, and in a 32-bit computer, 4 bytes are 1 word. On the premise of existence of the conversion relationship, the inventor considers that other units in other embodiments also belong to simple replacement of the solution of the present application and fall into the protection scope of the present application. In some specific embodiments of the present application, specified step lengths of a previous step of traversal and a latter step of traversal do not overlap.

For example, in the previous step of traversal, traversal is performed in the specified step length, such as 10 bytes, to identify whether there is the identifiable feature; and in the latter step of traversal, traversal is performed in the specified step length, such as 10 bytes, to identify whether there is the identifiable feature, but the 10 bytes of the latter step of traversal do not overlap the 10 bytes of the previous step of traversal.

For example, in the previous step of traversal, traversal is performed in the specified step length, such as 10 bytes, to identify whether there is the identifiable feature, in the latter step of traversal, the latter step of traversal is separated from the previous step of traversal by a certain distance, and then traversal is performed forwards or backwards in the specified step length, such as 10 bytes, to identify whether there is the identifiable feature, but the 10 bytes of the latter step of traversal do not overlap the 10 bytes of the previous step of traversal.

In some specific embodiments of the present application, a distance between the specified step lengths of the previous step of traversal and the latter step of traversal is greater than or equal to 1 byte.

For example, in the previous step of traversal, traversal is performed in the specified step length, such as 10 bytes, to identify whether there is the identifiable feature; and in the latter step of traversal, the latter step of traversal is separated from the previous step of traversal by 20 bytes, and then traversal is performed forwards or backwards in the specified step length, such as 10 bytes, to identify whether there is the identifiable feature.

The distance between the specified step lengths of the previous step of traversal and the latter step of traversal is fixed, and the distance is less than the size of 1 compression block.

For example, in the previous step of traversal, traversal is performed in the specified step length, such as 10 bytes, to identify whether there is the identifiable feature; and in the latter step of traversal, the latter step of traversal is separated from the previous step of traversal by a size of 0.8 compression block, and then traversal is performed forwards or backwards in the specified step length, such as 10 bytes, to identify whether there is the identifiable feature.

If the sizes of the compression blocks are far from each other, two identifiable features are identified in the compressed data, two corresponding headers are positioned according to the two identifiable features, the two headers are adjacent headers, a to-be-decompressed block is determined according to the two headers, and the to-be-decompressed block includes one compression block, the size of the compression block serves as the size of 1 compression block; or the sizes of the plurality of compression blocks are determined by referring to the above mode, and a mean value or median value of the sizes of any plurality of compression blocks serves as the size of 1 compression block. If the sizes of the compression blocks are similar, for some fields, such as the field of sequencing, it is known that sequencing data, such as fq data, is compressed into compressed data, the sizes of the compression blocks of the compressed data are all about 10 MB, and 10 MB may be determined as the size of 1 compression block, such that the size of 1 compression block is determined not by the above-mentioned mode for determining the to-be-decompressed block.

The distance is greater than or equal to the size of 0.5 compression blocks and less than the size of 1 compression block.

For example, the distance may be the size of 0.5 compression block, the size of 0.6 compression block, the size of 0.7 compression block, the size of 0.8 compression block, or the size of 0.9 compression block. Taking a numerical value, for example, the size of 1 compression block is 10 MB, and the distance may be 5 MB, 6 MB, 7 MB, 8 MB, or 9 MB.

In some specific embodiments of the present application, the distance between the specified step lengths of the previous step of traversal and the latter step of traversal is varied, and an initial distance may be set to be greater than or equal to the size of 5 compression blocks and less than or equal to the size of 15 compression blocks.

For example, in the previous step of traversal, traversal is performed in the specified step length, such as 10 bytes, to identify whether there is the identifiable feature; and in the latter step of traversal, the latter step of traversal is separated from the previous step of traversal by a size of 10 compression blocks, and then traversal is performed forwards or backwards in the specified step length, such as 10 bytes, to identify whether there is the identifiable feature.

If the sizes of the compression blocks are far from each other, two identifiable features are identified in the compressed data, two corresponding headers are positioned according to the two identifiable features, the two headers are adjacent headers, a to-be-decompressed block is determined according to the two headers, and the to-be-decompressed block includes one compression block, the size of the compression block serves as the size of 1 compression block; or the sizes of the plurality of compression blocks are determined by referring to the above mode, and a mean value or median value of the sizes of any plurality of compression blocks serves as the size of 1 compression block. If the sizes of the compression blocks are similar, for some fields, such as the field of sequencing, it is known that sequencing data, such as fq data, is compressed into compressed data, the sizes of the compression blocks of the compressed data are all about 10 MB, and 10 MB may be determined as the size of 1 compression block, such that the size of 1 compression block is determined not by the above-mentioned mode for determining the to-be-decompressed block.

Adaptively changing a distance between a next step of traversal and a current step of traversal according to the result of the current step of traversal further includes:
if the identifiable features are identified in the current step of traversal, jumping by the distance to perform the next step of traversal.

Specifically, if the identifiable features can be identified in the current step of traversal, it is known that the distance between the specified step lengths of the previous step of traversal and the latter step of traversal determined at this time is appropriate, the identifiable features can be identified with higher efficiency, and it continues to jump by the distance to perform the next step of traversal. In some specific embodiments of the present application, adaptively changing the distance between the next step of traversal and the current step of traversal according to the result of the current step of traversal further includes:
if the identifiable features are not identified in the current step of traversal, increasing the distance according to a preset rule, and then jumping by an increased distance to perform the next step of traversal.

Specifically, if the identifiable features are not identified in the current step of traversal, it is known that the distance between the specified step lengths of the previous step of traversal and the latter step of traversal determined at this time may be inappropriate, and the identifiable features cannot be identified with higher efficiency, the distance is increased according to the preset rule, and then it jumps by the increased distance to perform the next step of traversal.

The preset rule may be a preset multiple increase or exponential increase, or other rules with numerical value change.

For example, the preset multiple increase is a fixed multiple increase. For example, if the identifiable features are not identified in the current step of traversal, the distance is increased by 2 times, and then it jumps by the distance increased by 2 times to perform the next step of traversal. If the identifiable features are still not identified in the next step of traversal, the distance is still increased by 2 times, and then it jumps by the distance increased by 2 times to perform the next step of traversal. Taking a numerical value, for example, the distance is 10 MB, if the identifiable features are not identified in the current step of traversal, the distance is increased by 2 times, the distance is changed into 20 MB, it jumps by the distance of 20 MB to perform the next step of traversal, and if the identifiable features can be identified in the next step of traversal, the traversal is continued by taking 20 MB as the distance. If the identifiable features are still not identified in the next step of traversal, the distance is increased by 2 times, the distance is changed into 40 MB, it jumps by the distance of 40 MB to perform the next step of traversal, and if the identifiable features are identified in the next step of traversal, the traversal is continued by taking 40 MB as the distance.

For example, the exponential increase is an increase in multiples of 2, 4, 8, and the like. For example, if the identifiable features are not identified in the current step of traversal, the distance is increased by 2 times, and then it jumps by the distance increased by 2 times to perform the next step of traversal. If the identifiable features are not identified in the next step of traversal, the distance is increased by 4 times, and then it jumps by the distance increased by 4 times to perform the next step of traversal. Taking a numerical value, for example, the distance is 10 MB, if the identifiable features are not identified in the current step of traversal, the distance is increased by 2 times, the distance is changed into 20 MB, it jumps by the distance of 20 MB to perform the next step of traversal, and if the identifiable features can be identified in the next step of traversal, the traversal is continued by taking 20 MB as the distance. If the identifiable features are still not identified in the next step of traversal, the distance is increased by 4 times, the distance is changed into 80 MB, it jumps by the distance of 80 MB to perform the next step of traversal, and if the identifiable features are identified in the next step of traversal, the traversal is continued by taking 80 MB as the distance.

If the identifiable features are not identified in the current step of traversal, other ways may be adopted for the next step of traversal. For example, because there is a contingency in the current step of traversal, and it does not indicate that the distance used in the current step of traversal is definitely not the preferred distance, the distance remains unchanged, and it continues to jump by the distance to perform the next step of traversal. For example, the distance is shortened according to a preset rule, for example, the distance is shortened by a preset multiple or exponent, and then it jumps by the shortened distance to perform the next step of traversal.

If the method for decompressing data of the present application is applied to the field of sequencing, sequencing data such as fq sequencing data generated in the field of sequencing is compressed into compressed data, the compressed data includes a large quantity of compression blocks such as thousands of compression blocks, that is, a large quantity of headers such as thousands of headers are included, the compressed data is traversed in the specified step length, a certain distance is reserved between the specified step lengths of the previous step of traversal and the latter step of traversal, the time for traversing and identifying the headers can be effectively shortened, and thus the decompression efficiency is improved. In addition, the sequencing data is compressed into the compressed data, the sizes of the compression blocks included in the compressed data are close and are all about 10 MB, such that the distance between the specified step lengths of the previous step of traversal and the latter step of traversal can be more accurately determined in the decompression process, and it is ensured that the header can be positioned without taking a long time. If the sizes of the compression blocks in the compressed data are far from each other, for example, the size of one compression block is 20 MB, the size of another compression block is 500 MB, and multiple differences exist, there may be a case that the distance between the specified step lengths of the previous step of traversal and the latter step of traversal is not accurate and the header cannot be positioned quickly. In addition, the compressed data compressed by the sequencing data is very huge, usually tens of G, even hundreds of G, but the complete compressed data does not need to be decompressed, and usually only a fraction or a tenth of the compressed data needs to be decompressed, and the decompressed compressed data can meet the sequencing detection requirements. Therefore, the sizes of the determined to-be-decompressed blocks do not exceed the processing bearing range of a processor of a sequencing device, and thus the method for decompressing data of the present application can be applied to most sequencing devices on the market.

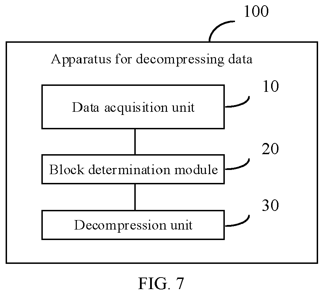

Referring to FIG. 7, an embodiment of the present disclosure provides an apparatus for decompressing data 100. The apparatus for decompressing data 100 includes a data acquisition unit 10, a block determination unit 20 and a decompression unit 30.

The data acquisition unit 10 is configured to perform a), acquiring compressed data, where the compressed data includes a plurality of compression blocks connected in series, one compression block includes a header and a data main body connected to the header, and the header has an identifiable feature; the block determination unit 20 is configured to perform b), identifying the identifiable feature to position a corresponding header, and determining one or more to-be-decompressed blocks in the compressed data according to the positioned header, where one to-be-decompressed block includes one or more compression blocks; and
the decompression unit 30 is configured to perform c), decompressing the to-be-decompressed block by a single thread or a plurality of threads so as to obtain decompressed compressed data,
where b) and c) are performed in parallel.

In the present embodiment, the data acquisition unit 10 is connected to the block determination unit 20, and the decompression unit 30 is connected to the block determination unit 20.

In the present embodiment, the compressed data may be in a format of rar, zip, gzip, etc., and the apparatus for decompressing data 100 of the present application is configured to decompress compressed data in various formats.

For example, compressed data in a gzip format is described in detail herein, the compressed data in the gzip format includes a plurality of compression blocks connected in series, each compression block includes a header and a data main body connected to the header, and at least part of the compression blocks may further include a tail portion connected to the data main body.

Regarding the compressed data in the gzip format, the compression blocks included therein are constructed as follows (see specifically https://blog.csdn.net/ljx333/article/details/77586147):

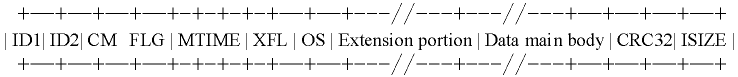

For each header, the header stores basic information of the compression block, including a data type, version, size, creation time, modification time, access right, etc., which has the function of helping an operating system identify the data type and access right. The header consists of a fixed-length portion and an extension portion, the fixed-length portion is the first ten bytes of the compression block, and the first ten bytes are as follows:
ID1 and ID2 each occupy 1 byte, and ID1 and ID2 are used to identify compression blocks in a gzip format, e.g., ID1 = 31 (0 × 1F), and ID2 = 139 (0 × 8B).

CM occupies 1 byte, and CM is used to identify a compression method used by a current compression block, e.g., CM = 8, indicating that the compression block employs a DEFLATE method.

FLG occupies 1 byte, and is a flag bit, where each bit in the flag bit represents whether a corresponding extension bit exists later, and the meaning of each bit is as follows: if the Bit 0FTEXT is set, it indicates that the compression block is ASCII text data; if Bit 1FHCRC is set, it indicates that the CRC16 check is performed on the header; if the Bit 2FEXTRA is set, it indicates that the extension portion exists; if the Bit 3FNAME is set, it indicates that a name field of compressed original data exists; if the Bit 4FCOMMENT is set, it indicates that a comment field exists; and Bits 5-7 are reserved and all set as 0.

MTIME occupies 4 bytes, and is used to represent the time when the compressed original data was last modified, the time being in a UINX format.

XFL occupies 1 byte, and is used to identify a compression method, and since the gzip format has only the DEFLATE method, aiming at the DEFLATE method, XFL has the following meaning: XFL = 2, which represents that the compression ratio is maximum but the compression speed is lowest; and XFL = 4, which represents that the compression speed is highest.

OS occupies 1 byte, and represents a file system to be compressed.

The fixed-length portion necessarily exists, the extension portion does not necessarily exist, and whether the extension portion exists or not is determined by the fixed-length portion. If the extension portion exists, the extension portion is divided into four parts, which are as follows in order (the order cannot be exchanged): FEXTRA + FNAME + FCOMMENT + FHCRC.

For the data main body, a quantity of bytes occupied by the data main body is determined according to the compressed original data.

For the tail portion, the tail portion occupies 4 bytes, CRC32 represents a result of calculating the compressed original data by using a standard redundancy check algorithm, and ISIZE represents a result of modulo 2^32 by the size of the compressed original data. In some embodiments, the compression block may contain only a header and a data main body, with no tail portion.

The thread is the smallest unit for the operation scheduling of the operating system, and belongs to the actual operation unit in a course. One thread refers to a single sequential control flow in the course, one course can concurrently run a plurality of threads, and each thread concurrently executes different tasks. According to the apparatus for decompressing data 100 of the present application, the to-be-decompressed block may be decompressed by the single thread, or the to-be-decompressed block may be decompressed by the plurality of threads, where a quantity of the plurality of threads is, for example, 4, 5, and 6, which is specifically configured according to actual situations, and is not limited herein.

According to the apparatus for decompressing data of the present application, specifically, the data acquisition unit 10 acquires the compressed data from a memory space, the compressed data includes the plurality of compression blocks connected in series, each compression block includes the header and the data main body connected to the header, and the header has the identifiable feature. For example, in the field of sequencing, a large amount of sequencing data such as fq data is compressed, in order to compress the data, and the compressed data may be obtained from the memory space, where the memory space may be a memory which is disposed inside a sequencer and used to store the compressed data or a memory which is connected to the sequencer externally and used to store the compressed data.

Then, b) is performed by the block determination unit 20, the identifiable feature is identified in the compressed data to position the corresponding header, and the specific identification process may start from the initial position of the compressed data and end at the middle position or the end position, and the corresponding header is positioned every time the feature the same as the identifiable feature is identified. After the header is positioned, one or more to-be-decompressed blocks in the compressed data are determined according to the header, where each to-be-decompressed block contains one or more compression blocks.

The middle position or the end position mentioned above is determined according to the requirement of decompressing the whole compressed data or part of the compressed data. For example, if the whole compressed data needs to be decompressed, the identification process starts from the initial position of the compressed data and ends at the end position; and if the part of the compressed data needs to be decompressed, the identification process starts from the initial position of the compressed data and ends at the middle position of the whole compressed data, and the middle position of the whole compressed data corresponds to the end position of the part of the compressed data needing to be decompressed. The middle position refers to any position from the initial position to the end position of the compressed data, and does not refer to a position of half the size of the compressed data.

In other embodiments, it may be realized that the compressed data is identified from a first middle position of the compressed data to a second middle position or the end position, and the identification process is as described above.

The identifiable feature consists of at least 1 byte, and identifying the identifiable feature refers to identifying the identifiable feature by identifying the at least 1 byte. If the identifiable feature consists of a plurality of bytes, the identifiable feature may be identified by identifying 1 byte in the plurality of bytes and further identifying the rest bytes; or the identifiable feature may be identified by identifying all of the plurality of bytes.

After one or more to-be-decompressed blocks are determined, c) is performed by the decompression unit 30, and the to-be-decompressed block is decompressed by the single thread or the plurality of threads so as to obtain the decompressed compressed data. A single thread decompression mode is adopted, and the determined to-be-decompressed blocks are decompressed one by one by the single thread. A multi-thread decompression mode is adopted, after the to-be-decompressed block is determined, if there is a thread in an idle state, the thread in the idle state is called to decompress the determined to-be-decompressed block, and if there is no thread in the idle state, it waits to call the thread in the idle state until there is the thread in the idle state, and then the thread in the idle state is called to decompress the determined to-be-decompressed block.

In the present embodiment, b) and c) mentioned above are performed in parallel.

Specifically, after one to-be-decompressed block or the plurality of to-be-decompressed blocks are determined, the thread in the idle state may be called to decompress the determined to-be-decompressed blocks, and compared to decompressing the to-be-decompressed blocks after all the to-be-decompressed blocks in the compressed data are determined, the decompression efficiency can be effectively improved.

For the apparatus for decompressing data, for example, the compressed data includes five compression blocks A, B, C, D, and E connected in series, and the plurality of threads are three threads a, b, and c. After the compressed data is obtained, starting from the initial position of the compressed data, a to-be-decompressed block A1 is determined, the to-be-decompressed block includes the compression block A, at this time, the threads a, b, and c are all in the idle state, and the thread a is called to decompress the to-be-decompressed block A1. Then a to-be-decompressed block B1 is determined, the to-be-decompressed block B1 includes the compression block B, at this time, the thread a is still decompressing the to-be-decompressed block A1, the threads B and c are both in the idle state, and the thread B is called to decompress the to-be-decompressed block B1. Then a to-be-decompressed block C1 is determined, the to-be-decompressed block C1 includes the compression block C, the thread a is still decompressing the to-be-decompressed block A1, the thread B is still decompressing the to-be-decompressed block B1, the thread C is in the idle state, and the thread C is called to decompress the to-be-decompressed block C1. Then a to-be-decompressed block D1 is determined, the to-be-decompressed block D1 includes the compression block D, at this time, the thread a completes decompression of the to-be-decompressed block A1 and is in the idle state, the thread B is still decompressing the to-be-decompressed block B1, the thread C is still decompressing the to-be-decompressed block C1, and the thread a is called to decompress the to-be-decompressed block D1. Then a to-be-decompressed block E1 is determined, the to-be-decompressed block E1 includes the compression block E, at this time, the thread b completes decompression of the to-be-decompressed block B1 and is in the idle state, the thread a is still decompressing the to-be-decompressed block D1, the thread c is still decompressing the to-be-decompressed block C1, and the thread b is called to decompress the to-be-decompressed block E1. After the thread a completes decompression of the to-be-decompressed block D1, the thread B completes decompression of the to-be-decompressed block E1 and the thread C completes decompression of the to-be-decompressed block C1, the to-be-decompressed blocks A1, B1, C1, D1, and E1 are all decompressed, such that the decompressed compressed data is obtained.

According to the apparatus for decompressing data 100 of the present application, the compressed data is obtained firstly, then the header of the compression block is positioned by identifying the identifiable feature, the to-be-decompressed block of the compressed data is determined according to the positioned header, and finally the determined to-be-decompressed block is decompressed by the single thread or the plurality of threads to obtain the decompressed compressed data. The data decompression process has high efficiency, and can effectively shorten the decompression time especially under the condition that huge data needs to be decompressed.

In some specific embodiments of the present application, the decompression unit 30 is further configured to connect, in series, the data after decompressing the to-be-decompressed block so as to obtain the decompressed compressed data.

Specifically, after the decompression of the to-be-decompressed blocks is completed, the data obtained by decompression is connected in series, such that the decompressed compressed data is obtained.

In some embodiments, every time decompression of one to-be-decompressed block is completed, the data obtained by decompression is connected according to a serial sequence. For example, a first to-be-decompressed block, a second to-be-decompressed block, and a third to-be-decompressed block are determined, and the serial sequence of the first to-be-decompressed block, the second to-be-decompressed block, and the third to-be-decompressed block is consistent with a serial sequence of the compression blocks contained therein. If the compression block contained in the second to-be-decompressed block is located between the compression block contained in the first to-be-decompressed block and the compression block contained in the third to-be-decompressed block in the serial sequence, after the first to-be-decompressed block and the second to-be-decompressed block are decompressed, the data obtained by decompression is connected in series, and after the third to-be-decompressed block is decompressed, the data after decompressing the third to-be-decompressed block is connected in series to the data connected in series after decompressing the first to-be-decompressed block and the second to-be-decompressed block. If the compression block contained in the third to-be-decompressed block is located between the compression block contained in the first to-be-decompressed block and the compression block contained in the second to-be-decompressed block in the serial sequence, the data obtained by decompression is connected in series according to the sequence of the first to-be-decompressed block, the third to-be-decompressed block, and the second to-be-decompressed block after the third to-be-decompressed block is decompressed even if the first to-be-decompressed block and the second to-be-decompressed block are decompressed firstly.

In some embodiments, after decompression of all the to-be-decompressed blocks is completed, the data obtained by decompression is connected according to the serial sequence. For example, decompression of all to-be-decompressed blocks such as the first to-be-decompressed block, the second to-be-decompressed block, and the third to-be-decompressed block is completed, and the serial sequence of the first to-be-decompressed block, the second to-be-decompressed block, and the third to-be-decompressed block is consistent with a serial sequence of the compression blocks contained therein. If the compression block contained in the second to-be-decompressed block is located between the compression block contained in the first to-be-decompressed block and the compression block contained in the third to-be-decompressed block in the serial sequence, the data obtained by decompression is connected in series according to the sequence of the first to-be-decompressed block, the second to-be-decompressed block, and the third to-be-decompressed block after decompression of the first to-be-decompressed block, the second to-be-decompressed block, and the third to-be-decompressed block is completed. If the compression block contained in the third to-be-decompressed block is located between the compression block contained in the first to-be-decompressed block and the compression block contained in the second to-be-decompressed block in the serial sequence, the data obtained by decompression is connected in series according to the sequence of the first to-be-decompressed block, the third to-be-decompressed block, and the second to-be-decompressed block after decompression of the first to-be-decompressed block, the second to-be-decompressed block, and the third to-be-decompressed block is completed.

In order to avoid the situation that the decompressed compressed data and the compressed original data have data non-correspondence, when c1) connecting, in series, data after decompressing the to-be-decompressed blocks is performed, no matter a mode that the data obtained by decompression is connected in series according to the serial sequence every time decompression of one to-be-decompressed block is completed, or a mode that the data obtained by decompression is connected in series according to the serial sequence after decompression of all the to-be-decompressed blocks is completed is adopted, the serial sequence is associated with the serial sequence of the compression blocks contained in the to-be-decompressed blocks, but not associated with a decompression completion sequence of the to-be-decompressed blocks. For example, the obtained compressed data includes the plurality of compression blocks A, B, C, D, and E connected in series, and then the to-be-decompressed blocks A1, B1, C1, D1, and E1 are determined from the compressed data, where the to-be-decompressed block A1 includes the compression block A, the to-be-decompressed block B1 includes the compression block B, the to-be-decompressed block C1 includes the compression block C, the to-be-decompressed block D1 includes the compression block D, and the to-be-decompressed block E1 includes the compression block E. After decompression of the to-be-decompressed blocks A1, B1, C1, D1, and E1 is completed, the data obtained by decompression is connected in series according to the sequence of the to-be-decompressed blocks A1, B1, C1, D1, and E1, and the decompressed compressed data is obtained.

The above series connection refers to that the initial position of the data obtained by decompressing a current to-be-decompressed block is connected to the end position of the data obtained by decompressing a previous to-be-decompressed block, and the initial position of the data obtained by decompressing a next to-be-decompressed block is connected to the end position of the data obtained by decompressing the current to-be-decompressed block. The initial position obtained by decompressing the first to-be-decompressed block according to the serial sequence is used as the initial position of the decompressed compressed data, and the end position obtained by decompressing the last to-be-decompressed block according to the serial sequence is used as the end position of the decompressed compressed data.

In some specific embodiments of the present application, the decompression unit 30 is further configured to decompress by the plurality of threads.

Specifically, after the to-be-decompressed blocks in the compressed data are determined, decompression is performed by the plurality of threads, decompression of the plurality of to-be-decompressed blocks can be performed simultaneously by using the plurality of threads, and the decompression efficiency is improved.

In some specific embodiments of the present application, the identifiable feature of the header is shown in the header as a portion with both size and structural features being fixed, such as the above fixed-length portion of the header, but the fixed-length portion of the header is a compression block of the compressed data corresponding to the gzip format, and for other formats, such as rar and zip formats, the identifiable feature of the header may be different from that in the gzip format. According to the apparatus for decompressing data 100 of the present application, the portion (such as the fixed-length portion in the gzip format) of the header is adopted as the identifiable feature, the header does not need to be modified, and the method is applied easily and conveniently and can be widely applied to various application environments.

The fixed portion consists of at least 1 byte, and can be identified by identifying the at least 1 byte. If the fixed portion consists of a plurality of bytes, the fixed portion may be identified by identifying 1 byte in the plurality of bytes and further identifying the rest bytes; or the fixed portion may be identified by identifying all of the plurality of bytes in the fixed portion.

The compressed data further has at least one of the following features (i) to (iii):
(i) the compressed data is in a gz format or a gzip format, and/or an extension name of the compressed data includes one of .GZIP, .gz, or .tgz, and/or the compressed data is obtained by compressing specified data through a DEFLATE algorithm-based compression algorithm, and/or the compressed data is obtained by compressing specified data through a gzip algorithm;
(ii) the portion is connected to the data main body; and/or optionally, the header further includes an extension portion connected to the portion, and the portion is connected to the data main body through the extension portion; and
(iii) the compression block further includes a tail portion connected to the data main body.

The DEFLATE algorithm mentioned herein is a lossless data compression algorithm composed of an LZ77 algorithm and Huffman Coding, and has been widely applied to processing of various storage data.

In some embodiments, the compressed data includes feature (i). In some embodiments, the compressed data includes feature (ii). In some embodiments, the compressed data includes feature (iii). In some embodiments, the compressed data includes features (i) and (ii). In some embodiments, the compressed data includes features (i) and (iii). In some embodiments, the compressed data includes features (ii) and (iii). In some embodiments, the compressed data includes features (i) to (iii).

In some embodiments, the identifiable feature of the header is not a portion of the header itself, but is an additionally added portion, for example, an additional byte is designed in the header as the identifiable feature, and the additional byte may be obtained by following a certain regular or irregular design, is distinguished from other bytes in the compression block, and can also be applied to the apparatus for decompressing data 100 of the present application.

In some specific embodiments of the present application, block compression is performed on the specified data so as to obtain the compressed data; optionally, block compression is performed by the DEFLATE algorithm-based compression algorithm, and the compression algorithm optionally is a gzip algorithm; and optionally, block compression is performed by the single thread or the plurality of threads.

Specifically, block compression is performed on the basis of the specified data, such as sequencing data, such that the compressed data is obtained, the compressed data including a plurality of compression blocks connected in series. After obtaining the compressed data, the above specified data is equal to the above compressed original data.

Block compression may be performed by the DEFLATE algorithm-based compression algorithm. The compression algorithm may be the gzip algorithm.

Block compression is performed by the single thread or the plurality of threads so as to obtain the compressed data. A single thread compression mode is adopted, and the specified data is compressed by the single thread. A multi-thread compression mode is adopted, if there is a thread in an idle state, the thread in the idle state is called to compress the specified data, and if there is no thread in the idle state, it waits to call the thread in the idle state until there is the thread in the idle state, and then the thread in the idle state is called to compress the specified data.

In some embodiments, according to the above-mentioned memory space, the compressed data obtained by block compression on the specified data may be stored in the memory space, and when the compressed data needs to be decompressed, the compressed data is obtained from the memory space.

Here, a method for compressing data is exemplified. Original data needing to be compressed is obtained firstly, then block processing is performed on the original data to obtain a plurality of blocks, then the blocks are compressed by the single thread or the plurality of threads to obtain a plurality of compression blocks, and the plurality of compression blocks are connected in series to generate the compressed data. The generated compressed data may be stored in the memory space. For example, in the field of sequencing, a large amount of sequencing data such as fq data is compressed, in order to compress the data, and the compressed data may be stored in the memory space, where the memory space may be a memory which is disposed inside a sequencer and used to store the compressed data or a memory which is connected to the sequencer externally and used to store the compressed data.

In some specific embodiments of the present application, the block determination unit 20 is configured to identify two identifiable features to position two corresponding headers, determine one to-be-decompressed block in the compressed data according to the two positioned headers, and enable the to-be-decompressed block to be subjected to single-thread or multi-thread decompression in the decompression unit 30.

Specifically, the block determination unit 20 identifies the two identifiable features in the compressed data, positions the two corresponding headers according to the two identifiable features, and then determines one to-be-decompressed block in the compressed data according to the two positioned headers, and the specific determining process is to determine the data between the two headers as one to-be-decompressed block, and then perform c) to decompress the to-be-decompressed block by the single thread or the plurality of threads. Every time b) is performed to determine one to-be-decompressed block, c) is performed to decompress the to-be-decompressed block, such that parallel execution of b) and c) is realized.

In some specific embodiments of the present application, the block determination unit 20 is further configured for the two positioned headers being adjacent headers in the compressed data. Specifically, the block determination unit 20 identifies the two identifiable features in the compressed data, positions the two corresponding headers according to the two identifiable features, the two positioned headers being adjacent headers in the compressed data, then determines one to-be-decompressed block in the compressed data according to the two positioned headers, the to-be-decompressed block including one compression block, and then perform c) to decompress the to-be-decompressed block by the single thread or the plurality of threads.

In the present embodiment, the compressed data is obtained, the compressed data includes the plurality of compression blocks connected in series, which are arranged in order as a first compression block, a second compression block, a third compression block, and so on. A first identifiable feature is identified in the compressed data, a corresponding first header is positioned according to the first identifiable feature, then a second identifiable feature is identified in the compressed data, a corresponding second header is positioned according to the second identifiable feature, the first header and the second header are adjacent headers, a first to-be-decompressed block is determined according to the first header and the second header, the first to-be-decompressed block includes the first compression block, and then the first to-be-decompressed block is decompressed by the single thread or the plurality of threads. A third identifiable feature is identified in the compressed data, a third header is positioned according to the third identifiable feature, the second header and the third header are adjacent headers, a second to-be-decompressed block is determined according to the second header and the third header, the second to-be-decompressed block includes the second compression block, and the second to-be-decompressed block is decompressed by the single thread or the plurality of threads. With reference to the above, the to-be-decompressed blocks are determined and decompressed until a last identifiable feature is identified (if the identifiable feature cannot be identified later, the identified identifiable feature is the last identifiable feature), a corresponding last header is positioned according to the last identifiable feature, a last to-be-decompressed block is determined according to the last header to the end position of the compressed data, the last to-be-decompressed block includes a last compression block, the last to-be-decompressed block is decompressed by the single thread or the plurality of threads, and finally the decompressed compressed data is obtained according to all the decompressed to-be-decompressed blocks.

According to the apparatus for decompressing data 100 of the present application, the to-be-decompressed block is determined by taking adjacent headers as the reference, and each to-be-decompressed block includes one compression block, thereby ensuring that the compression blocks in the compressed data are not missed and ensuring the integrity of the decompressed compressed data. Moreover, the data decompression process has high efficiency, and can effectively shorten the decompression time especially under the condition that huge data needs to be decompressed. For example, in the field of sequencing, sequencing data with a huge data size needs to be decompressed, and the decompression time can be effectively shortened by the apparatus for decompressing data 100. Moreover, in the field of sequencing, for sequencing data quickly generating a large amount of data in a short time or log data slowly generating a large amount of data in a long time, the apparatus for decompressing data 100 can effectively shorten the decompression time and also reduce the memory occupation.

In some specific embodiments of the present application, one or more headers exist between the two positioned headers.

Specifically, the block determination unit 20 identifies the two identifiable features in the compressed data, positions the two corresponding headers according to the two identifiable features, one or more headers existing between the two positioned headers, then determines one to-be-decompressed block in the compressed data according to the two positioned headers, the to-be-decompressed block including a plurality of compression blocks, and then perform c) to decompress the to-be-decompressed block by the single thread or the plurality of threads.

In the present embodiment, the compressed data is obtained, the compressed data includes the plurality of compression blocks connected in series. A first identifiable feature is identified in the compressed data, a corresponding first header is positioned according to the first identifiable feature, then a second identifiable feature is identified in the compressed data, a corresponding second header is positioned according to the second identifiable feature, one or more headers exist between the first header and the second header, a first to-be-decompressed block is determined according to the first header and the second header, the first to-be-decompressed block includes a plurality of compression blocks, and then the first to-be-decompressed block is decompressed by the single thread or the plurality of threads. A third identifiable feature is identified in the compressed data, a third header is positioned according to the third identifiable feature, one or more headers exist between the second header and the third header, a second to-be-decompressed block is determined according to the second header and the third header, the second to-be-decompressed block includes a plurality of compression blocks, and the second to-be-decompressed block is decompressed by the single thread or the plurality of threads. With reference to the above, the to-be-decompressed blocks are determined and decompressed until a last identifiable feature is identified (if the identifiable feature cannot be identified later, the identified identifiable feature is the last identifiable feature), a corresponding last header is positioned according to the last identifiable feature, a last to-be-decompressed block is determined according to the last header to the end position of the compressed data, the last to-be-decompressed block includes one or more compression blocks, the last to-be-decompressed block is decompressed by the single thread or the plurality of threads, and finally the decompressed compressed data is obtained according to all the decompressed to-be-decompressed blocks.

According to the apparatus for decompressing data 100 of the present application, the two corresponding headers are positioned according to the two identifiable features, one to-be-decompressed block in the compressed data is determined according to the two positioned headers, one or more headers exist between the two positioned headers, and the to-be-decompressed block includes the plurality of compression blocks. According to the apparatus for decompressing data 100, the plurality of compression blocks are combined into one to-be-decompressed block, and then the to-be-decompressed block is decompressed, such that the decompression efficiency can be further improved, and the decompression time can be effectively shortened especially under the condition that huge data needs to be decompressed. For example, in the field of sequencing, sequencing data with a huge data size needs to be decompressed, and the decompression time can be effectively shortened by the apparatus for decompressing data 100. Moreover, in the field of sequencing, for sequencing data quickly generating a large amount of data in a short time or log data slowly generating a large amount of data in a long time, the apparatus for decompressing data 100 can effectively shorten the decompression time and also reduce the memory occupation.

In some specific embodiments of the present application, the two headers are adjacent headers in the compressed data, the block determination unit 20 is further configured to identify another identifiable feature to position a corresponding header, and determine another to-be-decompressed block according to the header and one of adjacent headers relatively close to the header, and the another to-be-decompressed block includes a plurality of compression blocks.

Specifically, b) is performed firstly, the two identifiable features are identified in the compressed data, the two corresponding headers are positioned according to the two identifiable features, the two headers are adjacent headers, then one to-be-decompressed block is determined according to the two headers, the to-be-decompressed block includes one compression block, and then c) is performed to decompress the to-be-decompressed block by the single thread or the plurality of threads. b) is performed simultaneously with c) or after c), the another identifiable feature is identified, the corresponding header is positioned according to the another identifiable feature, the another to-be-decompressed block is determined according to the header and one of the two adjacent headers relatively close to the header, the another to-be-decompressed block includes a plurality of compression blocks, and then c) is performed to decompress the another to-be-decompressed block by the single thread or the plurality of threads.

In the present embodiment, b) is performed firstly, identifiable features A1 and B1 are identified in the compressed data, corresponding headers A2 and B2 are positioned according to the identifiable features A1 and B1, the headers A2 and B2 are adjacent headers, a to-be-decompressed block a is determined according to the headers A2 and B2, the to-be-decompressed block a includes one compression block, and then c) is performed to decompress the to-be-decompressed block a by the single thread or the plurality of threads. b2) is performed simultaneously with c) or after c), another identifiable feature C1 is identified, and a corresponding header C2 is positioned according to the identifiable feature C1. If the header C2 is located in front of the headers A2 and B2 in the compressed data (the header C2 is positioned in front of the headers A2 and B2), a distance between the header C2 and the header A2 is relatively shorter than a distance between the header C2 and the header B2, a to-be-decompressed block b1 is determined according to the header C2 and the header A2, the to-be-decompressed block b1 includes a plurality of compression blocks, and then c) is performed to decompress the to-be-decompressed block b1 by the single thread or the plurality of threads. If the header C2 is located behind the headers A2 and B2 in the compressed data (the header C2 is positioned behind the headers A2 and B2), a distance between the header C2 and the header B2 is relatively shorter than a distance between the header C2 and the header A2, a to-be-decompressed block b2 is determined according to the header B2 and the header C2, the to-be-decompressed block b2 includes a plurality of compression blocks, and then c) is performed to decompress the to-be-decompressed block b2 by the single thread or the plurality of threads.

According to the apparatus for decompressing data 100 of the present application, different identification results are integrated, different to-be-decompressed blocks are finally determined according to the different identification results, the different to-be-decompressed blocks have different quantities of compression blocks and each includes a single compression block or a plurality of compression blocks, and then the to-be-decompressed blocks are decompressed, such that the decompression efficiency can be further improved, and the decompression time can be effectively shortened especially under the condition that huge data needs to be decompressed. For example, in the field of sequencing, sequencing data with a huge data size needs to be decompressed, and the decompression time can be effectively shortened by the apparatus for decompressing data 100. Moreover, in the field of sequencing, for sequencing data quickly generating a large amount of data in a short time or log data slowly generating a large amount of data in a long time, the apparatus for decompressing data 100 can effectively shorten the decompression time and also reduce the memory occupation.

In some specific embodiments of the present application, the block determination unit 20 is further configured to traverse the compressed data in a specified step length to identify the identifiable features, the specified step length is greater than or equal to 1 byte and less than or equal to N times a size of the identifiable feature, and N is greater than or equal to 1.

Specifically, starting from the initial position of the compressed data, the identifiable features are identified in the specified step length, the corresponding header is positioned if the identifiable features are identified within the specified step length, and the header is not positioned if the identifiable features are not identified within the specified step length. The compressed data is traversed until to the end position of the compressed data needing to be decompressed. The size of the specified step length may or may not change in the traversal process.

It should be noted that, although the specified step length is less than or equal to N times the size of the identifiable feature, the specified step length is not calculated by the identifiable features, but the sizes of the specified step length and the identifiable features are only compared.

For example, starting from the initial position of the compressed data, 10 bytes are taken as the specified step length to identify the identifiable features, within each 10-byte range, whether the identifiable features are identified is judged, and if the identifiable features are identified, the corresponding header is positioned. The compressed data is traversed until to the end position of the compressed data needing to be decompressed. In the traversal process, the size of the specified step length may be changed to 9 bytes, 8 bytes or other bytes, or 10 bytes without change.

In the present embodiment, the specified step length is greater than or equal to 1 byte and less than or equal to N times the size of the identifiable feature. For example, for the compressed data in the gzip format, the size of the identifiable feature of the header of the compression block included in the compressed data is 10 bytes, so the specified step length may be defined to be greater than or equal to 1 byte and less than or equal to 10 bytes, and if N is 1, the specified step length is defined as 1 byte, 1.5 byte, 2 bytes, 2.5 bytes, 3 bytes, 3.5 bytes, 4 bytes, 4.5 bytes, 5 bytes, 5.5 bytes, 6 bytes, 6.5 bytes, 7 bytes, 7.5 bytes, 8 bytes, 8.5 bytes, 9 bytes, 9.5 bytes, or 10 bytes. The specified step length may be changed as long as the specified byte range is met.

In some specific embodiments of the present application, the specified step length is a fixed step length, and the fixed step length takes any integer value from 1 byte to N times the size of the identifiable feature. For example, for the compressed data in the gzip format, the size of the identifiable feature of the header of the compression block included in the compressed data is 10 bytes, so the specified step length may be defined to be greater than or equal to 1 byte and less than or equal to N times 10 bytes and be an integer value, and if N is 1, the specified step length is defined as 1 byte, 2 bytes, 3 bytes, 4 bytes, 5 bytes, 6 bytes, 7 bytes, 8 bytes, 9 bytes, or 10 bytes. Although the specified step length mentioned in the present application is in units of bytes, in other embodiments, the specified step length may be in other units, such as bits, and words. The conversion relationship is that 8 bits constitute 1 byte, in a 16-bit computer, 2 bytes are 1 word, and in a 32-bit computer, 4 bytes are 1 word. On the premise of existence of the conversion relationship, the inventor considers that other units in other embodiments also belong to simple replacement of the solution of the present application and fall into the protection scope of the present application. In some specific embodiments of the present application, specified step lengths of a previous step of traversal and a latter step of traversal do not overlap.

For example, in the previous step of traversal, traversal is performed in the specified step length, such as 10 bytes, to identify whether there is the identifiable feature; and in the latter step of traversal, traversal is performed in the specified step length, such as 10 bytes, to identify whether there is the identifiable feature, but the 10 bytes of the latter step of traversal do not overlap the 10 bytes of the previous step of traversal.

For example, in the previous step of traversal, traversal is performed in the specified step length, such as 10 bytes, to identify whether there is the identifiable feature, in the latter step of traversal, the latter step of traversal is separated from the previous step of traversal by a certain distance, and then traversal is performed forwards or backwards in the specified step length, such as 10 bytes, to identify whether there is the identifiable feature, but the 10 bytes of the latter step of traversal do not overlap the 10 bytes of the previous step of traversal.

In some specific embodiments of the present application, a distance between the specified step lengths of the previous step of traversal and the latter step of traversal is greater than or equal to 1 byte.

For example, in the previous step of traversal, traversal is performed in the specified step length, such as 10 bytes, to identify whether there is the identifiable feature; and in the latter step of traversal, the latter step of traversal is separated from the previous step of traversal by 20 bytes, and then traversal is performed forwards or backwards in the specified step length, such as 10 bytes, to identify whether there is the identifiable feature.

The distance between the specified step lengths of the previous step of traversal and the latter step of traversal is fixed, and the distance is less than the size of 1 compression block.

For example, in the previous step of traversal, traversal is performed in the specified step length, such as 10 bytes, to identify whether there is the identifiable feature; and in the latter step of traversal, the latter step of traversal is separated from the previous step of traversal by a size of 0.8 compression block, and then traversal is performed forwards or backwards in the specified step length, such as 10 bytes, to identify whether there is the identifiable feature.

If the sizes of the compression blocks are far from each other, two identifiable features are identified in the compressed data, two corresponding headers are positioned according to the two identifiable features, the two headers are adjacent headers, a to-be-decompressed block is determined according to the two headers, and the to-be-decompressed block includes one compression block, the size of the compression block serves as the size of 1 compression block; or the sizes of the plurality of compression blocks are determined by referring to the above mode, and a mean value or median value of the sizes of any plurality of compression blocks serves as the size of 1 compression block. If the sizes of the compression blocks are similar, for some fields, such as the field of sequencing, it is known that sequencing data, such as fq data, is compressed into compressed data, the sizes of the compression blocks of the compressed data are all about 10 MB, and 10 MB may be determined as the size of 1 compression block, such that the size of 1 compression block is determined not by the above-mentioned mode for determining the to-be-decompressed block.

The distance is greater than or equal to the size of 0.5 compression blocks and less than the size of 1 compression block.

For example, the distance may be the size of 0.5 compression block, the size of 0.6 compression block, the size of 0.7 compression block, the size of 0.8 compression block, or the size of 0.9 compression block. Taking a numerical value, for example, the size of 1 compression block is 10 MB, and the distance may be 5 MB, 6 MB, 7 MB, 8 MB, or 9 MB.

In some specific embodiments of the present application, the distance between the specified step lengths of the previous step of traversal and the latter step of traversal is varied, and an initial distance may be set to be greater than or equal to the size of 5 compression blocks and less than or equal to the size of 15 compression blocks.

For example, in the previous step of traversal, traversal is performed in the specified step length, such as 10 bytes, to identify whether there is the identifiable feature; and in the latter step of traversal, the latter step of traversal is separated from the previous step of traversal by a size of 10 compression blocks, and then traversal is performed forwards or backwards in the specified step length, such as 10 bytes, to identify whether there is the identifiable feature.

If the sizes of the compression blocks are far from each other, two identifiable features are identified in the compressed data, two corresponding headers are positioned according to the two identifiable features, the two headers are adjacent headers, a to-be-decompressed block is determined according to the two headers, and the to-be-decompressed block includes one compression block, the size of the compression block serves as the size of 1 compression block; or the sizes of the plurality of compression blocks are determined by referring to the above mode, and a mean value or median value of the sizes of any plurality of compression blocks serves as the size of 1 compression block. If the sizes of the compression blocks are similar, for some fields, such as the field of sequencing, it is known that sequencing data, such as fq data, is compressed into compressed data, the sizes of the compression blocks of the compressed data are all about 10 MB, and 10 MB may be determined as the size of 1 compression block, such that the size of 1 compression block is determined not by the above-mentioned mode for determining the to-be-decompressed block.

The block determination unit 20 is further configured to jump, if the identifiable features are identified in the current step of traversal, by the distance to perform the next step of traversal.

Specifically, if the identifiable features can be identified in the current step of traversal, it is known that the distance between the specified step lengths of the previous step of traversal and the latter step of traversal determined at this time is appropriate, the identifiable features can be identified with higher efficiency, and it continues to jump by the distance to perform the next step of traversal. In some specific embodiments of the present application, the block determination unit 20 is further configured to increase, if the identifiable features are not identified in the current step of traversal, the distance according to a preset rule, and then jump by an increased distance to perform the next step of traversal.

Specifically, if the identifiable features are not identified in the current step of traversal, it is known that the distance between the specified step lengths of the previous step of traversal and the latter step of traversal determined at this time may be inappropriate, and the identifiable features cannot be identified with higher efficiency, the distance is increased according to the preset rule, and then it jumps by the increased distance to perform the next step of traversal.

The preset rule may be a preset multiple increase or exponential increase, or other rules with numerical value change.

For example, the preset multiple increase is a fixed multiple increase. For example, if the identifiable features are not identified in the current step of traversal, the distance is increased by 2 times, and then it jumps by the distance increased by 2 times to perform the next step of traversal. If the identifiable features are still not identified in the next step of traversal, the distance is still increased by 2 times, and then it jumps by the distance increased by 2 times to perform the next step of traversal. Taking a numerical value, for example, the distance is 10 MB, if the identifiable features are not identified in the current step of traversal, the distance is increased by 2 times, the distance is changed into 20 MB, it jumps by the distance of 20 MB to perform the next step of traversal, and if the identifiable features can be identified in the next step of traversal, the traversal is continued by taking 20 MB as the distance. If the identifiable features are still not identified in the next step of traversal, the distance is increased by 2 times, the distance is changed into 40 MB, it jumps by the distance of 40 MB to perform the next step of traversal, and if the identifiable features are identified in the next step of traversal, the traversal is continued by taking 40 MB as the distance.

For example, the exponential increase is an increase in multiples of 2, 4, 8, and the like. For example, if the identifiable features are not identified in the current step of traversal, the distance is increased by 2 times, and then it jumps by the distance increased by 2 times to perform the next step of traversal. If the identifiable features are not identified in the next step of traversal, the distance is increased by 4 times, and then it jumps by the distance increased by 4 times to perform the next step of traversal. Taking a numerical value, for example, the distance is 10 MB, if the identifiable features are not identified in the current step of traversal, the distance is increased by 2 times, the distance is changed into 20 MB, it jumps by the distance of 20 MB to perform the next step of traversal, and if the identifiable features can be identified in the next step of traversal, the traversal is continued by taking 20 MB as the distance. If the identifiable features are still not identified in the next step of traversal, the distance is increased by 4 times, the distance is changed into 80 MB, it jumps by the distance of 80 MB to perform the next step of traversal, and if the identifiable features are identified in the next step of traversal, the traversal is continued by taking 80 MB as the distance.

If the identifiable features are not identified in the current step of traversal, other ways may be adopted for the next step of traversal. For example, because there is a contingency in the current step of traversal, and it does not indicate that the distance used in the current step of traversal is definitely not the preferred distance, the distance remains unchanged, and it continues to jump by the distance to perform the next step of traversal. For example, the distance is shortened according to a preset rule, for example, the distance is shortened by a preset multiple or exponent, and then it jumps by the shortened distance to perform the next step of traversal.

If the apparatus for decompressing data 100 of the present application is applied to the field of sequencing, sequencing data such as fq sequencing data generated in the field of sequencing is compressed into compressed data, the compressed data includes a large quantity of compression blocks such as thousands of compression blocks, that is, a large quantity of headers such as thousands of headers are included, the compressed data is traversed in the specified step length, a certain distance is reserved between the specified step lengths of the previous step of traversal and the latter step of traversal, the time for traversing and identifying the headers can be effectively shortened, and thus the decompression efficiency is improved. In addition, the sequencing data is compressed into the compressed data, the sizes of the compression blocks included in the compressed data are close and are all about 10 MB, such that the distance between the specified step lengths of the previous step of traversal and the latter step of traversal can be more accurately determined in the decompression process, and it is ensured that the header can be positioned without taking a long time. If the sizes of the compression blocks in the compressed data are far from each other, for example, the size of one compression block is 20 MB, the size of another compression block is 500 MB, and multiple differences exist, there may be a case that the distance between the specified step lengths of the previous step of traversal and the latter step of traversal is not accurate and the header cannot be positioned quickly. In addition, the compressed data compressed by the sequencing data is very huge, usually tens of G, even hundreds of G, but the complete compressed data does not need to be decompressed, and usually only a fraction or a tenth of the compressed data needs to be decompressed, and the decompressed compressed data can meet the sequencing detection requirements. Therefore, the sizes of the determined to-be-decompressed blocks do not exceed the processing bearing range of a processor of a sequencing device, and thus the apparatus for decompressing data 100 of the present application can be applied to most sequencing devices on the market.

An embodiment of the present disclosure provides an electronic device, including a memory, configured to store data and including a computer executable program; and a processor, configured to execute the computer executable program to implement the method for decompressing data mentioned above.

According to the electronic device for decompressing data of the present application, compressed data is obtained firstly, then a header of a compression block is positioned by identifying an identifiable feature, a to-be-decompressed block of the compressed data is determined according to the positioned header, and finally the determined to-be-decompressed block is decompressed by a single thread or a plurality of threads so as to obtain decompressed compressed data. The data decompression process has high efficiency, and can effectively shorten the decompression time especially under the condition that huge data needs to be decompressed.

An embodiment of the present disclosure provides a computer-readable storage medium, configured to store a program executed by a computer, where executing the program includes implementing the method for decompressing data mentioned above.

According to the computer-readable storage medium of the present application, compressed data is obtained firstly, then a header of a compression block is positioned by identifying an identifiable feature, a to-be-decompressed block of the compressed data is determined according to the positioned header, and finally the determined to-be-decompressed block is decompressed by a single thread or a plurality of threads so as to obtain decompressed compressed data. The data decompression process has high efficiency, and can effectively shorten the decompression time especially under the condition that huge data needs to be decompressed.

An embodiment of the present disclosure provides a sequencer, including the above-mentioned electronic device; or the above-mentioned storage medium.

According to the sequencer of the present application, compressed data is obtained firstly, then a header of a compression block is positioned by identifying an identifiable feature, a to-be-decompressed block of the compressed data is determined according to the positioned header, and finally the determined to-be-decompressed block is decompressed by a single thread or a plurality of threads so as to obtain decompressed compressed data. The data decompression process has high efficiency, and can effectively shorten the decompression time especially under the condition that huge data needs to be decompressed.

The above embodiments only illustrate one or more embodiments of the present application for the purpose of specific and detailed description, but should not be construed as limiting the scope of the present application. It should be noted that various changes and modifications can be made by those of ordinary skill in the art without departing from the ideas of the present application, and these changes and modifications are all within the protection scope of the present application. Therefore, the protection scope of the present application should be determined with reference to the appended claims.

## Claims

1. A method for decompressing data, comprising:
a) acquiring compressed data, wherein the compressed data comprises a plurality of compression blocks connected in series, one compression block comprises a header and a data main body connected to the header, and the header has an identifiable feature;
b) identifying the identifiable feature to position a corresponding header, and determining one or more to-be-decompressed blocks in the compressed data according to the positioned header, wherein one to-be-decompressed block comprises one or more compression blocks; and
c) decompressing the to-be-decompressed block by a single thread or a plurality of threads so as to obtain decompressed compressed data,
wherein b) and c) are performed in parallel.

2. The method for decompressing data according to claim 1, wherein c) further comprises connecting, in series, data after decompressing the to-be-decompressed block so as to obtain the decompressed compressed data;
wherein optionally decompression is performed by the plurality of threads.

3. The method for decompressing data according to claims 1 or 2, wherein the identifiable feature of the header is shown in the header as a portion with both size and structural features being fixed; and
optionally, the compressed data further has at least one of the following features (i) to (iii),
(i) the compressed data is in a gzip format, and/or an extension name of the compressed data comprises one of .GZIP, .gz, or .tgz, and/or the compressed data is obtained by compressing specified data through a DEFLATE algorithm-based compression algorithm, and/or the compressed data is obtained by compressing specified data through a gzip algorithm;
(ii) the portion is connected to the data main body; and/or optionally, the header further comprises an extension portion connected to the portion, and the portion is connected to the data main body through the extension portion; and
(iii) the compression block further comprises a tail portion connected to the data main body;
wherein optionally block compression is performed on the specified data so as to obtain the compressed data; optionally, block compression is performed by the DEFLATE algorithm-based compression algorithm, and the compression algorithm optionally is a gzip algorithm; and optionally, block compression is performed by the single thread or the plurality of threads.

4. The method for decompressing data according to claim 3, wherein b) comprises identifying two identifiable features to position two corresponding headers, determining one to-be-decompressed block in the compressed data according to the two positioned headers, and enabling the to-be-decompressed block to be subjected to single-thread or multi-thread decompression of c);
wherein optionally i) the two positioned headers are adjacent headers in the compressed data; or
ii) wherein one or more headers exist between the two positioned headers; or
iii) wherein the two headers are adjacent headers in the compressed data, b) further comprises identifying another identifiable feature to position a corresponding header, and determining another to-be-decompressed block according to the header and one of adjacent headers relatively close to the header, and the another to-be-decompressed block comprises a plurality of compression blocks.

5. The method for decompressing data according to claim 4, wherein the compressed data is traversed in a specified step length to identify the identifiable features, the specified step length is greater than or equal to 1 byte and less than or equal to N times a size of the identifiable feature, and N is greater than or equal to 1; wherein optionally the specified step length is a fixed step length, and the fixed step length takes any integer value from 1 byte to N times the size of the identifiable feature.

6. The method for decompressing data according to claim 5, wherein specified step lengths of a previous step of traversal and a latter step of traversal do not overlap;
wherein optionally a distance between the specified step lengths of the previous step of traversal and the latter step of traversal is greater than or equal to 1 byte; and optionally, the distance between the specified step lengths of the previous step of traversal and the latter step of traversal is fixed, the distance is less than a size of 1 compression block, and optionally the distance is greater than or equal to a size of 0.5 compression block and less than the size of 1 compression block;
wherein further optionally the distance between the specified step lengths of the previous step of traversal and the latter step of traversal is varied, and an initial distance is set to be greater than or equal to a size of 5 compression blocks and less than or equal to a size of 15 compression blocks; and optionally, adaptively changing a distance between a next step of traversal and a current step of traversal according to a result of the current step of traversal, comprises:
if the identifiable features are identified in the current step of traversal, jumping by the distance to perform the next step of traversal;
wherein further optionally adaptively changing the distance between the next step of traversal and the current step of traversal according to the result of the current step of traversal further comprises: if the identifiable features are not identified in the current step of traversal, increasing the distance according to a preset rule, and then jumping by an increased distance to perform the next step of traversal.

7. An apparatus for decompressing data, comprising:
a data acquisition unit, configured to perform a), acquiring compressed data, wherein the compressed data comprises a plurality of compression blocks connected in series, one compression block comprises a header and a data main body connected to the header, and the header has an identifiable feature;
a block determination unit, configured to perform b), identifying the identifiable feature to position a corresponding header, and determining one or more to-be-decompressed blocks in the compressed data according to the positioned header, wherein one to-be-decompressed block comprises one or more compression blocks; and
a decompression unit, configured to perform c), decompressing the to-be-decompressed block by a single thread or a plurality of threads so as to obtain decompressed compressed data,
wherein b) and c) are performed in parallel.

8. The apparatus for decompressing data according to claim 7, wherein the decompression unit is further configured to connect, in series, data after decompressing the to-be-decompressed block so as to obtain the decompressed compressed data;
wherein optionally the decompression unit is further configured to perform decompression by the plurality of threads.

9. The apparatus for decompressing data according to claims 7 or 8, wherein the identifiable feature of the header is shown in the header as a portion with both size and structural features being fixed; and optionally, the compressed data further has at least one of the following features (i) to (iii),
(i) the compressed data is in a gz format or a gzip format, and/or an extension name of the compressed data comprises one of .GZIP, .gz, or .tgz, and/or the compressed data is obtained by compressing specified data through a DEFLATE algorithm-based compression algorithm, and/or the compressed data is obtained by compressing specified data through a gzip algorithm;
(ii) the portion is connected to the data main body; and/or optionally, the header further comprises an extension portion connected to the portion,
and the portion is connected to the data main body through the extension portion; and
(iii) the compression block further comprises a tail portion connected to the data main body;
wherein optionally block compression is performed on the specified data so as to obtain the compressed data; optionally, block compression is performed by the DEFLATE algorithm-based compression algorithm, and the compression algorithm optionally is a gzip algorithm; and optionally, block compression is performed by the single thread or the plurality of threads.

10. The apparatus for decompressing data according to claim 9, wherein the block determination unit is configured to identify two identifiable features to position two corresponding headers, determine one to-be-decompressed block in the compressed data according to the two positioned headers, and enable the to-be-decompressed block to be subjected to single-thread or multi-thread decompression in the decompression unit;
wherein optionally i) the two positioned headers are adjacent headers in the compressed data; or
ii) wherein one or more headers exist between the two positioned headers; or
iii) wherein the two headers are adjacent headers in the compressed data, the block determination unit is further configured to identify another identifiable feature to position a corresponding header, and determine another to-be-decompressed block according to the header and one of adjacent headers relatively close to the header, and the another to-be-decompressed block comprises a plurality of compression blocks.

11. The apparatus for decompressing data according to claim 10, wherein the block determination unit is further configured to traverse the compressed data in a specified step length to identify the identifiable features, the specified step length is greater than or equal to 1 byte and less than or equal to N times a size of the identifiable feature, and N is greater than or equal to 1;
wherein optionally the specified step length is a fixed step length, and the fixed step length takes any integer value from 1 byte to N times the size of the identifiable feature.

12. The apparatus for decompressing data according to claim 11, wherein specified step lengths of a previous step of traversal and a latter step of traversal do not overlap;
wherein optionally a distance between the specified step lengths of the previous step of traversal and the latter step of traversal is greater than or equal to 1 byte; and optionally, the distance between the specified step lengths of the previous step of traversal and the latter step of traversal is fixed, the distance is less than a size of 1 compression block, and optionally the distance is greater than or equal to a size of 0.5 compression block and less than the size of 1 compression block.

13. The apparatus for decompressing data according to claim 12, wherein the distance between the specified step lengths of the previous step of traversal and the latter step of traversal is varied, and an initial distance is set to be greater than or equal to a size of 5 compression blocks and less than or equal to a size of 15 compression blocks; and optionally, the block determination unit is further configured to jump, if the identifiable features are identified in a current step of traversal, by the distance to perform a next step of traversal;
wherein optionally the block determination unit is further configured to, if the identifiable features are not identified in the current step of traversal, increase the distance according to a preset rule, and then jump by an increased distance to perform the next step of traversal.

14. An electronic device, comprising a memory, configured to store data and comprising a computer executable program; and a processor, configured to execute the computer executable program to implement the method for decompressing data as defined in any one of claims 1 to 6.

15. A computer-readable storage medium, configured to store a program executed by a computer, wherein executing the program comprises implementing the method for decompressing data as defined in any one of claims 1 to 6.
